(19) Europäisches Patentamt
European Patent Office
Office européen des brevets

(11) Veröffentlichungsnummer: **0 200 685**
**B1**

(12) # EUROPÄISCHE PATENTSCHRIFT

(45) Veröffentlichungstag der Patentschrift: **06.12.89**

(51) Int. Cl.⁴: **C 07 C 67/03**, C 07 C 69/732, C 07 C 69/88

(21) Anmeldenummer: **86810190.8**

(22) Anmeldetag: **28.04.86**

(54) **Verfahren zur Herstellung von sterisch gehinderten Hydroxyphenylcarbonsäureestern.**

(30) Priorität: **02.05.85 CH 1870/85**

(43) Veröffentlichungstag der Anmeldung: **05.11.86 Patentblatt 86/45**

(45) Bekanntmachung des Hinweises auf die Patenterteilung: **06.12.89 Patentblatt 89/49**

(84) Benannte Vertragsstaaten: **CH DE FR GB IT LI NL**

(56) Entgegenhaltungen:
**EP-A-0 102 920**
**EP-A-0 148 729**

(73) Patentinhaber: **CIBA- GEIGY AG, Klybeckstrasse 141, CH- 4002 Basel (CH)**

(72) Erfinder: **Orban, Ivan, Dr., Lehenmattstrasse 216, CH- 4052 Basel (CH)**

**EP 0 200 685 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Herstellung von sterisch gehinderten Hydroxyphenylcarbonsäureestern, bei welchem eine Umesterung durch Behandlung mit einem organischen oder anorganischen Zinksalz durchgeführt und die erhaltene Schmelze in einer Kurzzeit-Destillationsapparatur destilliert wird.

Umesterungsreaktionen zur Herstellung von sterisch gehinderten Hydroxyphenylcarbonsäureestern sind bekannt. So beschreiben z. B. die DE-AS-1 201 349 und die DE-OS-1 543 644 Umesterungsreaktionen dieser Art, bei welchen Alkalialkoholate als Katalysatoren eingesetzt werden. Umesterungsreaktionen der gleichen Art werden nach der DE-OS-2 150 327 mit Lithiumamid katalysiert. Bei all diesen Verfahren entstehen in mehr oder weniger kleinen Mengen Nebenprodukte (meistens Oxydationsprodukte von 2,6-Dialkylphenolen), die selbst in sehr geringen Mengen eine drastische Herabsetzung der Lagerstabilität des gewünschten Endproduktes verursachen. Die unumgängliche Entfernung dieser Nebenprodukte ist zeit-, arbeits- und energieaufwendig. In EP-A-102 920 wird zur Lösung dieses Problems vorgeschlagen die Umesterung durch sukzessive Behandlung mit katalytischen Mengen einer metallorganischen Verbindung von Metallen der vierten Haupt- oder Nebengruppe des Periodensystems und einer sauren Erde zu katalysieren.

Es wurde nun gefunden, dass die Durchführung der Umesterung ohne Lösungsmittel und ohne Verwendung von sauren Erden in Gegenwart katalytischer Mengen eines organischen oder anorganischen Zinksalzes mit anschliessender Destillation der erhaltenen Schmelze in einer Kurzzeit-Destillataionsapparatur bei bestimmten Bedingungen überraschenderweise zu einer praktisch quantitativen Ausbeute an reinem Endprodukt führt, das, da keine störenden Nebenprodukte enthaltend, nicht zusätzlich gereinigt werden muss. Ein weiterer Vorteil dieses Verfahrens ist die lösungsmittelfreie Arbeitsweise.

Die vorliegende Erfindung betrifft demnach ein Verfahren zur Herstellung von Verbindungen der Formel I

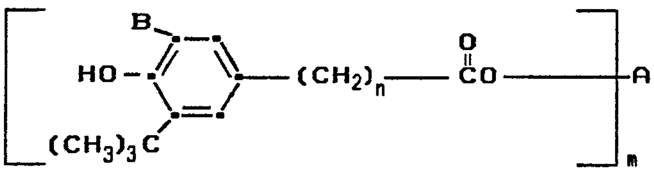

(I)

worin n die Zahlen 0 bis 2, m die Zahlen 1 oder 2, A einen von einem m-wertigen aliphatischen Alkohol abgeleiteten Rest mit 2 bis 18 Kohlenstoffatomen und B Methyl oder t-Butyl bedeuten, durch Umesterung von ca. m Molen und, wenn m > 1 ist, mit einem Überschuss bis 15 Mol.-%, eines Esters der Formel II

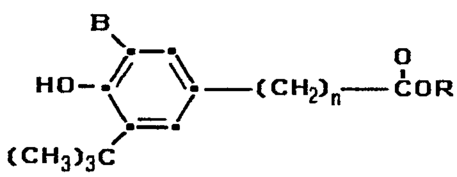

(II)

worin R Methyl oder Ethyl bedeutet, mit einem Alkohol der Formel (III)

$A{-}({-}OH)_m$ (III)

dadurch gekennzeichnet, dass

a) die Umesterung in Gegenwart eines organischen oder anorganischen Zinksalzes als Katalysator in einer Menge zwischen 0,05 und 2,0 Mol.-% bezogen auf den Ester der Formel II durchgeführt und
b) die erhaltene Schmelze in einer Kurzzeit-Destillationsapparatur bei einem Druck zwischen 0,5 und 6 mbar, bevorzugt zwischen 1 und 3 mbar und bei einer Temperatur zwischen 230 und 270°C, bevorzugt zwischen 240 und 260°C andestilliert und die anfallende Schmelze granuliert wird und
c) die Umesterung ohne Lösungsmittel durchgeführt wird.

A ist als ein von einem m-wertigen aliphatischen Alkohol abgeleiteter Rest ein m-wertiger substituierter oder unsubstituierter aliphatischer Rest mit 2 bis 18 Kohlenstoffatomen.

Ist m 1, so kann es sich bei A um $C_2$-$C_{18}$-Alkyl, geradkettig oder verzweigt handeln, wie z. B. Ethyl, n-Propyl, Isopropyl, n-Butyl, sec.-Butyl, tert.-Butyl, n-Pentyl, n-Hexyl, 2-Ethylhexyl, n-Octyl, 1,1,3,3-Tetramethylbutyl, n-Decyl, n-Dodecyl, n-Hexadecyl oder n-Octadecyl. Bevorzugt ist n-Octadecyl.

Ist m 2, so kann A beispielsweise $C_2$-$C_{18}$-Alkylen, bevorzugt $C_2$-$C_6$-Alkylen sein, wie Dimethylen, Trimethylen, Tetramethylen, Hexamethylen, 2,2-Dimethyl-trimethylen, Octamethylen, Nonamethylen, Decamethylen, Dodecamethylen oder Octadecamethylen. Die Alkylengruppe kann unterbrochen sein durch -O-, -S- oder -N(R)- wie in 2-Thiapropylen-1,3, 3-Thiapentylen-1,5, 4-Oxaheptamethylen, 3,6-Dioxaoctamethylen-1,8 oder 3,6-Diazaoctamethylen-1,8. Ist A unterbrochenes $C_2$-$C_6$-Alkylen, so ist es bevorzugt eine Gruppe

$-(CH_2)_2-S-(CH_2)_2$ oder $-(CH_2)_2-O-(CH_2)_2-O-(CH_2)_2$.

Geeignete Zinksalze als Katalysatoren sind z. B. Zinkchlorid, Zinksulfat, Zinkstearat und insbesondere Zinkacetat, wasserfrei oder mit Kristallwasser, z. B. als Dihydrat.

Der Katalysator wird bevorzugt in Mengen zwischen 0,1 und 0,5 Mol.-% bezogen auf den Ester der Formel II, bei Temperaturen zwischen 110 und 220°C, besonders bevorzugt zwischen 140 und 190°C und bei einem Druck zwischen 1000 und 2, bevorzugt zwischen 250 und 3 mbar, während 2 bis 5 Stunden eingesetzt.

Bei Verbindungen der Formel I, worin m 1 ist, ist es zweckmässig einen Überschuss von 1 bis 15 Mol.-% an Ester der Formel II einzusetzen.

Geeignete Kurzzeit-Destillationsapparaturen sind z. B. Filmtruder, Fallfilmverdampfer und insbesondere Dünnschichtverdampfer. Die Destillation wird vorteilhaft kontinuierlich durchgeführt, jedoch ist eine diskontinuierliche Arbeitsweise auch möglich.

Die aus der Kurzzeit-Destillation erhaltene Schmelze braucht nur noch nach üblichen Methoden granuliert zu werden, z. B. auf dem Kühlband, um das fertige Endprodukt in praktisch reiner, freifliessender, staubfreier, für dessen spezifischen Einsatz bereiter Form zu erhalten. Das erfindungsgemässe Verfahren ist besonders geeignet für die Herstellung von Verbindungen der Formel I durch Umesterung eines Esters der Formel II mit einem Alkohol der Formel III, worin in den Formeln I, II und III

n die Zahl 2 und m die Zahlen 1 oder 2,
A bei m = 1 $C_2$-$C_{18}$-Alkyl,
A bei m = 2 eine Gruppe
$-(CH_2)_2-O-(CH_2)_2-O-(CH_2)_2-$ und
R Methyl bedeuten.

Bevorzugtes erfindungsgemässes Verfahren ist die Herstellung von Octadecyl-3-(3,5-di-tert.-butyl-4-hydroxyphenyl)-propionat durch Umesterung von Methyl-3-(3,5-di-tert.-butyl-4-hydroxyphenyl)-propionat mit Stearylalkohol, dadurch gekennzeichnet, dass

a) die Umesterung in Gegenwart von Zinkacetat durchgeführt und
b) die erhaltene Schmelze in einer Kurzzeit-Destillationsapparatur bei einem Druck zwischen 1 und 3 mbar und bei einer Temperatur zwischen 240 und 270°C andestilliert und die anfallende Schmelze granuliert wird.

Bei den Estern der Formel II, den Alkoholen der Formel III und den Katalysatoren handelt es sich um bekannte Substanzen. Sollten einzelne dieser Substanzen neu sein, so können sie nach allgemein bekannten Verfahren hergestellt werden.

Die Verbindungen der Formel I sind wertvolle Stabilisatoren für organische Materialien, die der Zersetzung unterworfen sind, zum Beispiel für synthetische organische Polymere, tierische und pflanzliche Öle, Kohlenwasserstoffe, Schmiermittel und dergleichen.

Die folgenden Beispiele erläutern die Erfindung, ohne sie einzuschränken.

**Beispiel 1**

a) In einem Kolben, welcher mit einem auf 80°C beheizten Dephlegmator versehen ist, werden 1051 g (3,6 Mol) Methyl-3-(3,5-di-tert.-butyl-4-hydroxyphenyl)-propionat und 1001 g (3,7 Mol) Stearylalkohol vorgelegt und auf 80°C erwärmt. Es werden dann 1,35 g (0,007 Mol) Zinkacetat wasserfrei zugegeben und unter Stickstoff bei Normaldruck auf 175°C erhitzt. Das entstehende Methanol wird dabei in der Vorlage abdestilliert. Nun wird der Kolben bei 175°C, innerhalb einer Stunde, stufenweise auf 1 bis 3 mbar evakuiert und dabei die Methanol-Destillation fortgesetzt.

Zum Schluss wird für eine weitere Stunde bei 175°C und 1 bis 3 mbar gerührt.

b) Die erhaltene leicht gelbliche Schmelze wird kontinuierlich durch einen Dünnschichtverdampfer gelassen (Druck 1 bis 3 mbar, Innentemperatur 240 bis 270°C). Im unteren Teil des Verdampfers wird die Produkteschmelze gekühlt und verlässt den Verdampfer mit einer Temperatur von 60 bis 80°C. Anschliessend wird sie auf ein Blech gegossen und nach dem Erstarren zerkleinert.

Unter Betriebsbedingungen wird die Schmelze direkt zur Granulierung geführt.

Man erhält 1900 g (99,5 % d. Th.) Octadecyl-3-(3,5-di-tert.-butyl-4-hydroxyphenyl)-propionat mit Smp. 50 bis 51°C.

**Beispiel 2**

Das Verfahren des Beispiels 1 wird wiederholt mit der einzigen Ausnahme, dass statt 1001 g nur 972 g (3,6 Mol) Stearylalkohol eingesetzt werden.

Man erhält 1895 g (99,3 % d. Th.) Octadecyl-3-(3,5-di-tert.-butyl-4-hydroxyphenyl)-propionat mit Smp. 50 bis 51°C.

**Beispiel 3**

Das Verfahren des Beispiels 1 wird wiederholt mit der Ausnahme, dass die Umesterung bei 160°C, statt bei 175°C durchgeführt wird und die Nachrührzeit bei 1 bis 3 mbar 2 Stunden, anstatt einer Stunde dauert. Man erhält das Produkt von Beispiel 1 mit gleichem Schmelzpunkt und gleicher Ausbeute.

**Beispiel 4**

Das Verfahren des Beispiels 1 wird wiederholt mit der Ausnahme, dass als Katalysator 1,6 g (0,007 Mol) Zinkacetat-Dihydrat eingesetzt wird, und das Reaktionsgemisch nach Zugabe des Katalysators zuerst 15 Minuten bei 80°C/1 – 3 mb gerührt (Entfernung des Hydratwassers) und erst dann bei Normaldruck auf 175°C Temperatur aufgeheizt wird. Man erhält das Produkt von Beispiel 1 mit gleichem Schmelzpunkt und gleicher Ausbeute.

## Patentansprüche

1. Verfahren zur Herstellung von Verbindungen der Formel I

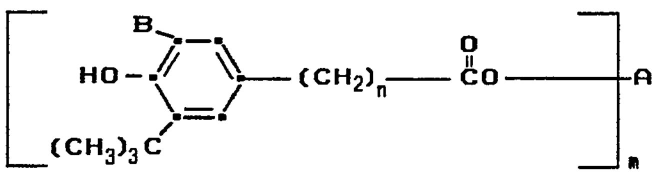

(I)

worin n die Zahlen 0 bis 2, m die Zahlen 1 oder 2, A einen von einem m-wertigen aliphatischen Alkohol abgeleiteten Rest mit 2 bis 18 Kohlenstoffatomen und B Methyl oder t-Butyl bedeuten, durch Umesterung von ca. m Molen und, wenn m> 1 ist, mit einem Überschuss bis 15 Mol.-% eines Esters der Formel II

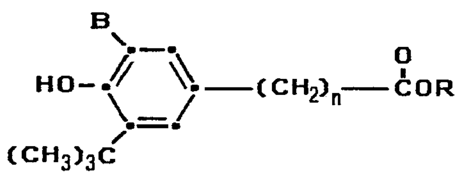

(II),

worin R Methyl oder Ethyl bedeutet, mit einem Alkohol der Formel (III)

$A-(OH)_m$ (III)

dadurch gekennzeichnet, dass
a) die Umesterung in Gegenwart eines organischen oder anorganischen Zinksalzes als Katalysator in einer Menge zwischen 0,05 und 2,0 Mol.-% bezogen auf den Ester der Formel II durchgeführt und
b) die erhaltene Schmelze in einer Kurzzeit-Destillationsapparatur bei einem Druck zwischen 0,5 und 6 mbar und bei einer Temperatur zwischen 230 und 270°C andestilliert und die anfallende Schmelze granuliert wird und
c) die Umesterung ohne Lösungsmittel durchgeführt wird.

2. Verfahren gemäss Anspruch 1, dadurch gekennzeichnet, dass als Katalysator Zinkacetat eingesetzt wird.

3. Verfahren gemäss Anspruch 1 zur Herstellung von Verbindungen der Formel I durch Umesterung eines Esters der Formel II mit einem Alkohol der Formel III, wobei in den Formeln I, II und III

n die Zahl 2 und 0 die Zahlen 1 oder 2,
A bei m = 1 $C_2$-$C_{18}$-Alkyl,
A bei m = 2 eine Gruppe
$-(CH_2)_2-O-(CH_2)_2-O-(CH_2)_2-$
und
R Methyl bedeuten.

4. Verfahren gemäss Anspruch 1, dadurch gekennzeichnet, dass bei der Umesterung der Katalysator in Mengen zwischen 0,1 und 0,5 Mol.-% bezogen auf den Ester der Formel II, bei Temperaturen zwischen 110 und 220°C und bei einem Vakuum zwischen 1000 und 2 mbar eingesetzt wird.

5. Verfahren gemäss Anspruch 1, dadurch gekennzeichnet, dass die Destillation der Schmelze in der Kurzzeit-Destillationsapparatur bei einem Druck zwischen 1 und 3 mbar und bei einer Temperatur zwischen 240 und 260°C durchgeführt wird.

6. Verfahren gemäss Anspruch 1, zur Herstellung von Octadecyl-3(3,5-di-tert.-butyl-4-hydroxyphenyl)-propionat durch Umesterung von Methyl-3-(3,5-di-tert.-butyl-4-hydroxyphenyl)-propionat mit Stearylalkohol.

## Claims

1. A process for the preparation of a compound of formula I

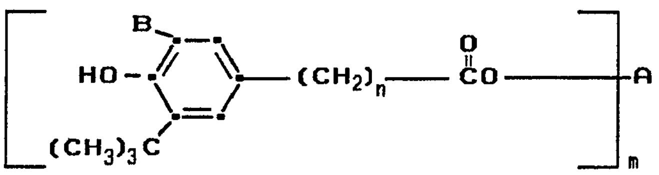

(I)

in which n is the number from 0 to 2, m is the numbers 1 or 2, A is a radical derived from an m-valent aliphatic alcohol, which radical contains 2 to 18 carbon atoms, and B is methyl or tert.-butyl, by transesterification of about m moles and, if m > 1, with an excess of up to 15 mol.-%, of an ester of formula II

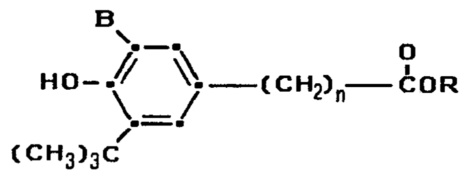

(II)

in which R is methyl or ethyl, with an alcohol of formula (III)

$A-(OH)_m$ (III)

which process comprises

a) carrying out the transesterification in the presence of an organic or inorganic zinc salt in an amount between 0.05 and 2.0 mol.-%, based on the ester of formula II, and

b) subjecting the resultant melt to incipient distillation in a short-time distillation apparatus under a pressure between 0.5 and 6 mbar and at a temperature between 230 and 270°C, and granulating the melt obtained, and

c) carrying out the transesterification without a solvent.

2. A process according to claim 1, in which the catalyst employed is zinc acetate.

3. A process according to claim 1 for the preparation of a compound of formula I by transesterification of an ester of formula II with an alcohol of formula III, in which process, in formulae I, II and III,

n is the number 2 and m is the number 1 or 2,
A is $C_2$-$C_{18}$ alkyl if m is 1,
A is a $-(CH_2)_2-O-(CH_2)_2-O-(CH_2)_2-$ if m is 2,
and
R is methyl.

4. A process according to claim 1, which comprises employing the catalyst in the transesterification in amounts between 0.1 and 0.5 mol.-%, based on the ester of formula II, at temperatures between 110 and 220°C and under a vacuum between 10000 and 2 mbar.

5. A process according to claim 1, which comprises carrying out the distillation of the melt in the short-time distillation apparatus under a pressure between 1 and 3 mbar and at a temperature between 240 and 260°C.

6. A process according to claim 1 for the preparation of octadecyl 3(3,5-di-tert.-butyl-4-hydroxyphenyl)propionate by transesterification of methyl 3-(3,5-di-tert.-butyl-4-hydroxyphenyl)propionate with stearyl alcohol.

**Revendications**

1. Procédé pour préparer des composés répondant à la formule I:

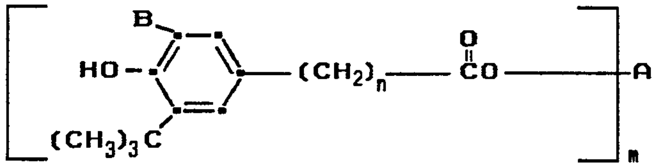

(I)

dans laquelle n désigne un nombre de 0 à 2, m désigne le nombre 1 ou le nombre 2, A représente un radical qui dérive d'un alcool aliphatique de fonctionnalité égale à m et qui contient de 2 à 18 atomes de carbone, et B représente un radical méthyle ou tert.-butyle, par transestérification d'environ m moles, et, lorsque m est supérieur à 1, avec un excès d'au plus 15 % en moles, d'un ester répondant à la formule II:

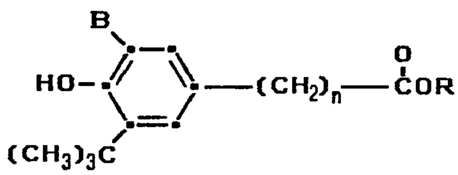

(II),

dans laquelle R représente un radical méthyle ou éthyle, avec un alcool de formule III:

$A-(OH)_m$ (III)

procédé caractérisé en ce que:

a) on effectue la transestérification en présence d'un sel de zinc minéral ou organique comme catalyseur en une quantité comprise entre 0,05 et 2,0 % en moles par rapport à l'ester de formule II,

b) on distille le mélange fondu obtenu dans un appareil de distillation rapide sous une pression comprise entre 0,5 et 6 mbar, de préférence entre 1 et 3 mbar, et à une température comprise entre 230 et 270°C, de préférence entre 240 et 260°C, et on granule le produit fondu obtenu, et

c) on effectue la transestérification sans solvant.

2. Procédé selon la revendication 1 caractérisé en ce qu'on utilise de l'acétate de zinc comme catalyseur.

3. Procédé selon la revendication 1 pour préparer des composés de formule I par transestérification d'un ester de formule II avec un alcool de formule III dans lesquels

n désigne le nombre 2, m désigne le nombre 1 ou le nombre 2,

A représente un alkyle en $C_2$-$C_{18}$ lorsque m est égal à 1 ou un radical:

$-(CH_2)_2-O-(CH_2)_2-O-(CH_2)_2-$

lorsque m est égal à 2,

et

R représente un radical méthyle.

4. Procédé selon la revendication 1 caractérisé en ce que le catalyseur est utilisé, lors de la transestérification, en des quantités comprises entre 0,1 et 0,5 % en moles par rapport à l'ester de formule II, à des températures comprises entre 110 et 220°C et sous un vide compris entre 1000 et 2 mbar.

5. Procédé selon la revendication 1 caractérisé en ce que la distillation du mélange fondu est exécutée dans un appareil de distillation rapide, sous une pression comprise entre 1 et 3 mbar et à une température comprise entre 240 et 260°C.

6. Procédé selon la revendication 1 pour préparer le (di-tert.-butyl-3,5 hydroxy-4 phényl)-3 propionate d'octadécyle par transestérification du (di-tert.-butyl-3,5 hydroxy-4 phényl)-3 propionate de méthyle avec l'alcool stéarylique.